# EUROPÄISCHE PATENTSCHRIFT

(11) **EP 1 434 840 B1**
(45) Veröffentlichungstag und Bekanntmachung des Hinweises auf die Patenterteilung: **24.11.2010**
(21) Anmeldenummer: 02785155.9
(22) Anmeldetag: 04.10.2002
(51) Int. Cl.: C11D 1/94, C11D 3/20

(54) **ANIONTENSIDFREIE NIEDRIGVISKOSE TRÜBUNGSMITTEL**
LOW-VISCOSITY OPACIFIERS FREE FROM ANIONIC SURFACE-ACTIVE AGENTS
OPACIFIANTS DE FAIBLE VISCOSITE, EXEMPTS DE TENSIOACTIFS ANIONIQUES

(30) Priorität: 13.10.2001 DE 10150725
(43) Veröffentlichungstag der Anmeldung: 07.07.2004
(73) Patentinhaber: Cognis IP Management GmbH, 40589 Düsseldorf (DE)
(72) Erfinder: NIEENDICK, Claus, 47807 Krefeld (DE); KOESTER, Josef, 40221 Düsseldorf (DE); KUBILK, Heike, 47906 Kempen (DE)
(86) Internationale Anmeldenummer: PCT/EP2002/011105
(87) Internationale Veröffentlichungsnummer: WO 2003/033634

(56) Entgegenhaltungen:
- WO-A-02/05781
- WO-A-99/09944
- DE-A- 19 937 298
- DE-C- 19 725 964

## Beschreibung

### Gebiet der Erfindung

Die Erfindung betrifft Trübungsmittel mit einem Gehalt an Wachskörpem, amphoteren Tensiden, Fettsäurepartialglyceriden, Fettsäurepolyglycolestem und gegebenenfalls Polyolen in einem bestimmten Gewichtsverhältnis und ohne Anwesenheit von Aniontensiden sowie deren Verwendung als Trübungsmittel.

### Stand der Technik

Bei der Formulierung einer Vielzahl von oberflächenaktiven Haushaltsprodukten wie beispielsweise Geschirrspülmitteln oder kosmetischen Zubereitungen, wie beispielsweise Haarshampoos wird besonderer Wert darauf gelegt, daß die Produkte möglichst klar vorliegen und auch im Verlauf der Lagerung nicht austrüben. In anderen Fällen werden für den gleichen Anwendungszweck Produkte gewünscht, die trübe sind und dabei einen Glimmereffekt, den sogenannten "Perlglanz" zeigen. Eine dritte Gruppe von Produkten wird mit einer nichtglänzenden Weißtrübung hergestellt, wobei sogenannte Trübungsmittel zum Einsatz gelangen.

Trübungsmittel stellen feinteilige Polymer- bzw. Feststoffdispersionen dar, die neben Wasser und/oder einem Polyol - beispielsweise Glycerin - im wesentlichen nur noch einen Wachskörper und einen geeigneten Emulgator enthalten. Die aus dem Stand der Technik bekannten Trübungsmittel basieren hauptsächlich auf Copolymerisaten auf Basis von Acyl- bzw. Methacrylsäure und Styrol und sind nicht biologisch abbaubar. Aus dem Deutschen Patent DE 19511572 C2 sind niedrigviskose Trübungsmittelkonzentrate aus Basis von Wachskörpern, Zuckertensiden und Partialglyceriden bekannt. Diese Konzentrate zeigen zwar gute biologische Abbaubarkeiten, weisen jedoch hohe Viskositäten auf und sind hinsichtlich der Feinteiligkeit verbesserungswürdig. Des Weiteren zeigen Formulierungen mit Zuckertensiden eine geringere Lagerstabilität bei höheren Temperaturen, dies betrifft insbesondere die Farbqualität und -stabilität.

Demzufolge hat die Aufgabe der Erfindung demnach darin bestanden, Trübungsmittelzubereitungen bzw. Konzentrate auf Basis von Wachskörpem zur Verfügung zu stellen, die hochkonzentriert sind, aber im Vergleich zum Stand der Technik deutlich verminderte durchschnittliche Teilchengrößen aufweisen. Sie sollten niedrigviskoser sein, um sich leicht verarbeiten zu lassen und biologisch abbaubar sein. Weiterhin sollten diese Zubereitungen in wäßrigen Tensidlösungen eine verstärkte Weißtrübung und keinen Perlglanz hervorrufen und infolge ihrer Teilchengrösse auch bei erhöhten Temperaturen ausreichend lagerstabil sein.

### Beschreibung der Erfindung

Gegenstand der Erfindung sind Trübungsmittelzubereitungen auf Basis von Wachskörpern, enthaltend
(a) mindestens ein amphoteres Tensid,
(b) mindestens ein Fettsäurepartialglycerid,
(c) mindestens ein Fettsäurepolyglycolester und gegebenenfalls
(d) ein Polyol
   mit der Massgabe, dass die Gewichtsverhältnisse von (a) : (b) zwischen 10 : 1 und 4 : 1 und (b) :
(c) zwischen 1 : 1 und 5 :1 1 liegen und die Zubereitungen frei von Aniontensiden sind.

In einer besonderen Ausführungsform der Erfindung liegt das Gewichtsverhältnis der Komponenten (a) : (b) zwischen 7 : 1 und 5 :1 1 und (b) : (c) zwischen 2 : 1 und 4: 1.

Überraschenderweise konnte gefunden werden, daß Mischungen auf Basis von Wachskörpem mit amphoteren Tensiden, Partialglyceriden, Fettsäurepolyglycolestern und gegebenenfalls Polyolen in einem ausgewählten Gewichtsverhältnis Produkte ergeben, die im Vergleich zum Stand der Technik eine besonders kleine durchschnittliche Teilchengrösse aufweisen. Demzufolge wird die gewünschte Weisstrübung ebenfalls durch diese besonders feinteiligen Zubereitungen verstärkt und es entsteht kein Perlglanz. Darüber hinaus sind diese Produkte besonders niedrig viskos, biologisch abbaubar, zeigen gute Fliess- und Pumpeigenschaften, sind auch bei Temperaturen oberhalb von 30°C ausreichend lagerstabil und kompatibel mit kationischen Formulierungkomponenten. Diese vorteilhaften Eigenschaften können nur für aniontensidfreie Trübunsmittelsysteme erreicht werden.

### Amphotere Tenside

Die erfindungsgemäßen Zubereitungen können amphotere Tenside enthalten, wie beispielsweise Alkylbetaine, Alkylamidobetaine, Aminopropionate, Aminoglycinate, Imidazoliniumbetaine und Sulfobetaine. Vorzugsweise werden Betaine eingesetzt. Betaine stellen bekannte Tenside dar, die überwiegend durch Carboxyalkylierung, vorzugsweise Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Ferner ist auch die Anlagerung von ungesättigten Carbonsäuren, wie beispielsweise Acrylsäure möglich. Zur Nomenklatur und insbesondere zur Unterscheidung zwischen Betainen und "echten" Amphoteren Tensiden sei auf den Beitrag von U. Ploog in Seifen-Öle-Fette-Wachse, 108, 373 (1982**)** verwiesen. Weitere Übersichten zu diesem Thema finden sich beispielsweise von A.O'Lennick et al. in HAPPI, Nov. 70 (1986**),** S.Holzman et al. in Tens. Surf.Det. 23, 309 (1986**),** R.Bibo et al. in Soap Cosm.Chem.Spec., Apr. 46 (1990**)** und P.Ellis et al. in Euro Cosm. 1, 14 (1994). Beispiele für geeignete Betaine stellen die Carboxyalkylierungsprodukte von sekundären und insbesondere tertiären Aminen dar, die der Formel **(I)** folgen, 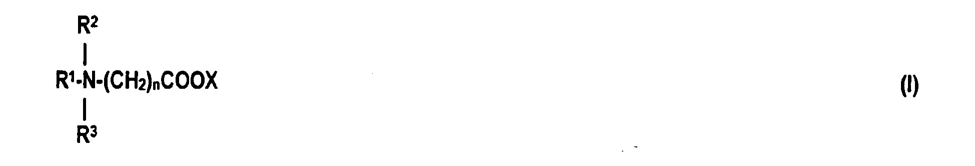 in der R¹ für Alkyl- und/oder Alkenylreste mit 6 bis 22 Kohlenstoffatomen, R² für Wasserstoff oder Alkylreste mit 1 bis 4 Kohlenstoffatomen, R³ für Alkylreste mit 1 bis 4 Kohlenstoffatomen, n für Zahlen von 1 bis 6 und X für ein Alkali- und/oder Erdalkalimetall oder Ammonium steht.

Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Weiterhin kommen auch Carboxyalkylierungsprodukte von **Amidoaminen** in Betracht, die der Formel (11) folgen, 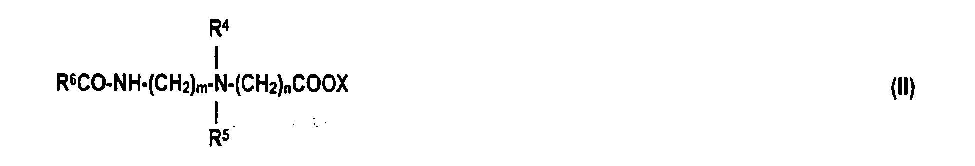 in der R⁶CO für einen aliphatischen Acylrest mit 6 bis 22 Kohlenstoffatomen und 0 oder 1 bis 3 Doppelbindungen, m für Zahlen von 1 bis 3 steht, R⁴ für Wasserstoff oder Alkylreste mit 1 bis 4 Kohlenstoffatomen, R⁵ für Alkylreste mit 1 bis 4 Kohlenstoffatomen, n für Zahlen von 1 bis 6 und X für ein Alkali-und/oder Erdalkalimetall oder Ammonium steht.

Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat.

Weiterhin kommen als geeignete Ausgangsstoffe für die im Sinne der Erfindung einzusetzenden Betaine auch Imidazoline in Betracht, die der Formel (III) folgen, 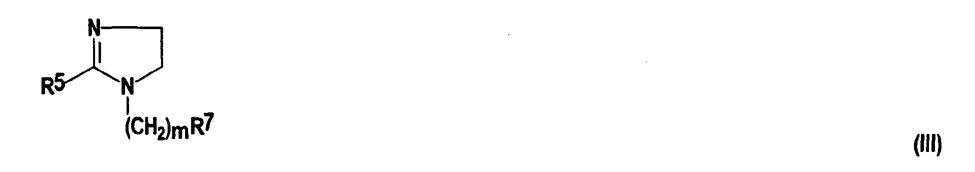 in der R⁷ für einen Alkylrest mit 5 bis 21 Kohlenstoffatomen, R⁶ für eine Hydroxylgruppe, einen OCOR⁵-oder NHCOR⁵-Rest und m für 2 oder 3 steht. Auch bei diesen Substanzen handelt es sich um bekannte Stoffe, die beispielsweise durch cyclisierende Kondensation von 1 oder 2 Mol Fettsäure mit mehrwertigen Aminen, wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum C_{12/14}-Kokosfettsäure, die anschließend mit Natriumchloracetat betainisiert werden.

Besonders geeignete Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Hierunter bevorzugt ist das unter der CTFA-Bezeichnung *Cocamidopropyl Betaine* bekannte Fettsäureamid-Derivat. Besonders bevorzugt sind Betaine, die sich durch eine hohe Reinheit auszeichnen, dieses bedeutet, es werden salzarme Betaine eingesetzt, deren Gehalt an Salzen maximal 13 Gew. %, bevorzugt maximal 11 Gew. % und besonders bevorzugt maximal 7 Gew. % bezogen auf die Aktivsubstanz ausmacht. Das entsprechende Salz ist von der Herstellung des Amphoteren Tensides abhängig, im häufigsten Fall handelt es sich dabei um Natriumchlorid. Speziell bevorzugt haben diese Betaine auch einen geringen Gehalt an freien Fettsäuren von maximal 4 Gew. % , bevorzugt maximal 3 Gew. % bezogen auf die Aktivsubstanz.

Die erfindungsgemäßen Zubereitungen können die amphoteren Tenside in Mengen von 1 bis 25, vorzugsweise 5 bis 20 und insbesondere 10 bis 15 Gew. % - bezogen auf die Endzusammensetzung - enthalten.

### Fettsäurepartialglyceride

Fettsäurepartialglyceride, also Monoglyceride, Diglyceride und deren technische Gemische können herstellungsbedingt noch geringe Mengen Di und Triglyceride enthalten. Die Partialglyceride folgen vorzugsweise der Formel **(IV),** 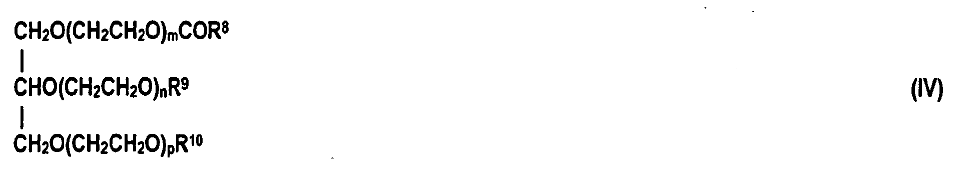 in der R⁸CO für einen linearen oder verzweigten, gesättigten und/oder ungesättigten Acylrest mit 6 bis 22, vorzugsweise 12 bis 18 Kohlenstoffatomen, R⁹ und R¹⁰ unabhängig voneinander für R⁸CO oder OH und die Summe (m+n+p) für 0 oder Zahlen von 1 bis 100, vorzugsweise 5 bis 25 steht, mit der Maßgabe, daß mindestens einer der beiden Reste R⁹ und R¹⁰ OH bedeutet. Typische Beispiele sind Mono-und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden technische Laurinsäureglyceride, Palmitinsäureglyceride, Stearinsäureglyceride, Isostearinsäureglyceride, Ölsäureglyceride, Behensäureglyceride und/oder Erucasäureglyceride eingesetzt, welche einen Monoglyceridanteil im Bereich von 50 bis 95, vorzugsweise 60 bis 90 Gew.-% aufweisen. Insbesondere werden längerkettige Partialglyceride z.B. basierend auf Ölsäure oder Stearinsäure eingesetzt insbesondere Gemische von Glyceriden auf Basis von gesättigten und ungesättigten Fettsäuren.

Die erfindungsgemäßen Zubereitungen können die Fettsäurepartialglyceride in Mengen von 0,1 bis 6, vorzugsweise 0,5 bis 5 und insbesondere 1 bis 3 Gew.-% - bezogen auf die Endzusammensetzung - enthalten.

### Fettsäurepolyglycolester

Als Fettsäurepolyglycolester werden bevorzugt Polyethylenglycol-Glycerylfettsäureester von Fettsäuren mit 8 bis 18-Kohlenstoffatomen und 3 bis 40, bevorzugt 4 bis 10 Polyethylenglycoleinheiten eingesetzt. Die erfindungsgemäßen Zubereitungen können die Fettsäurepolyglycolester in Mengen von 0,1 bis 6, vorzugsweise 0,2 bis 2 und insbesondere 0,5 bis 1 Gew.-% - bezogen auf die Endzusammensetzung - enthalten.

### Polyole

Polyole, die als fakultative Komponente im Sinne der Erfindung in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Die bevorzugt eingesetzten Polyole sind Glycerin und Propylenglycol, sie werden in Mengen von 0 bis 10 Gew. %, vorzugsweise 0,1 bis 8 Gew. % und besonders bevorzugt 1 bis 5 Gew. % - bezogen auf die Endzusammensetzung - verwendet.

### Wachskörper

Die Auswahl der Wachskörper ist an sich unkritisch. Typische Beispiele sind Alkylenglycolfettsäureester, Wachsester, gehärtete Triglyceride, gesättigte Fettalkohole mit 16 bis 18 Kohlenstoffatomen, Ethylenoxid-Addukte an Fettsäuren mit 16 bis 18 Kohlenstoffatomen und/oder Paraffinwachse.

### Alkylenglycolfettsäureester

In einer weiteren bevorzugten Ausführungsform der Erfindung werden als Wachskörper Alkylenglycolfettsäureestern der Formel **(V)** eingesetzt,

R¹¹CO-O-[A]-O-R¹² (V)

in der R¹¹CO für einen aliphatischen Acylrest mit 6 bis 22 Kohlenstoffatomen und 0 und/oder 1, 2 oder 3 Doppelbindungen, R¹² für R¹¹CO oder eine Hydroxylgruppe und A für eine lineare oder verzweigte, gegebenenfalls hydroxysubstituierte Alkylen- gruppe mit 2 bis 5 Kohlenstoffatomen steht.

Vorzugsweise handelt es sich bei diesen Wachsen um Ester des Ethylenglycols oder Propylenglycols mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Ara- chinsäure, Gadoleinsäure, Behensäure und Erucasäure sowie deren technische Mischungen. Besonders bevorzugt ist der Ein- satz von Ethylenglycoldistearat.

Die erfindungsgemäßen Zubereitungen können die Wachskörper, vorzugsweise Alkylenglycolfettsäureester in Mengen von 10 bis 35, vorzugsweise 12 bis 28 und insbesondere 15 bis 25 Gew.-% - bezogen auf die Endzusammensetzung - enthalten.

### Gewerbliche Anwendbarkeit

Die erfindungsgemäßen Trübungsmittelzubereitungen weisen vorzugsweise einen Feststoffgehalt von 20 bis 50 und besonders bevorzugt 30 bis 45 und insbesondere 35 bis 42 Gew.-% - bezogen auf die Endzusammensetzung - auf. Ein weiterer Gegenstand der Erfindung betrifft daher deren Verwendung als Trübungsmittel, vorzugsweise in kosmetischen Zubereitungen.

Sie zeichnen sich durch niedrige Viskositäten, vorzugsweise von 2000 bis 6000 und insbesondere 3000 bis 5000 mPas (nach Brookfield: 23 °C, Spindel 5, 10 Upm), gute Fließ- und Pumpeigenschaften sowie eine besondere Feinteiligkeit der Kristalle in der Dispersion aus. Die besondere Feinteiligkeit wird durch eine Partikelgrössenverteilung erzeugt, bei der mindestens 85, vorzugsweise 90 und besonders bevorzugt 95 und insbesondere 99,9 % der Teilchen einen Durchmesser von <15 µm ausmachen. Der durchschnittliche Teilchendurchmesser ist dabei vorzugsweise < 12, besonders bevorzugt < 10 und insbesondere < 7 µm. Für das Trübungsverhalten und die Partikelverteilung erwies sich die Auswahl von Fettsäurepartialglyceriden mit einem Schmelzpunkt von oberhalb 30°C und Fettsäurepolyolestern mit einem Schmelzpunkt unterhalb von 25 °C als besonders geeignet.

Ein weiterer Vorteil dieser Mittel besteht in ihrer hohen Stabilität gegen Sedimentieren bei längerer Lagerung. Die erfindungsgemäßen Trübungsmittelzubereitungen werden in Mengen von 0,1 bis 12, vorzugsweise 0,5 bis 6 und insbesondere 1 bis 3,5 Gew.-% - bezogen auf die wäßrigen oberflächenaktive Mittel, wie beispielsweise manuelle Wasch- und Reinigungsmittel, kosmetischen und/oder pharmazeutischen Zubereitungen, wie beispielsweise Haarshampoos, Haarlotionen, Schaumbäder, Duschbäder, Mund- und Zahnpflegemittel, Cremes, Gele, Lotionen, wäßrig/alkoholische Lösungen, Emulsionen und dergleichen - eingesetzt, rufen die Mittel eine dauerhafte, gleichmäßige und im Vergleich zum Stand der Technik eine besonders intensive Weißtrübung hervor, ohne daß dabei Perlglanz erzeugt wird.

Die Auswahl der Tenside, in deren wäßrigen Lösungen die erfindungsgemäßen Mittel eine Weißtrübung hervorrufen, ist hierbei sehr wichtig, da der Zusatz von Aniontensiden eine deutliche Erhöhung der Viskosität hervorruft und die gewünschte Feinteiligkeit und damit die besonders intensive Weisstrübung ausbleibt. Hier kommt es dann auch meist zur Ausbildung von Perlglanz da durch die Verwendung von Aniontensiden auch grössere Partikel vorhanden sind. Demzufolge können die Trübungsmittel lediglich in wäßrigen Lösungen von nichtionischen und/oder amphoteren bzw. zwitterionischen Tensiden eingesetzt werden. Vorzugsweise werden sie in wässrigen Lösungen mit amphoteren bzw. zwitterionischen Tensiden eingesetzt.

Die erfindungsgemäßen Tensidgemische können ferner als weitere Hilfs- und Zusatzstoffe weitere Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können nichtionische und/oder kationische Tenside enthalten sein. Typische Beispiele für nichtionische Tenside sind Fettalkoholpolyglycolether, Alkylphenolpolyglycolether, Fettsäureamidpolyglycolether, Fettaminpolyglycolether, alkoxylierte Triglyceride, Mischether bzw. Mischformale, gegebenenfalls partiell oxidierte Glucoronsäurederivate, Fettsäure-N-alkylglucamide, Proteinhydrolysate (insbesondere pflanzliche Produkte auf Weizenbasis), Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217 verwiesen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 A1), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren (vgl. EP 97/00434), Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 **PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 **PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet und folgen der allgemeinen Formel 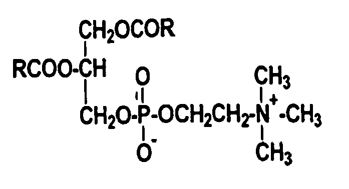 wobei R typischerweise für lineare aliphatische Kohlenwasserstoffreste mit 15 bis 17 Kohlenstoffatomen und bis zu 4 cis-Doppelbindungen steht. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, VinylpyrrolidonNinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, VinylpyrrolidonNinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in Cosmetics & Toiletries Vol. 108, Mai 1993, Seite 95ff aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976**).**

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**)** sowie Parfümerie und Kosmetik 3 (1999), Seite 11ff zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Trüsopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R. Lochhead in Cosm.Toil. 108, 95 (1993**)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon,Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Das Wachs, die Fettsäurepolyolester (c), Partialglyceride (b) und die amphoteren Tenside (a) wurden in unterschiedlichen Gewichtsverhältnissen gemischt und die Feinteiligkeit über Bestimmung der Partikelgrößenverteilung in µm und dem durchschnittlichen Teilchendurchmesser in µm mittels Laserbeugung (Mastersizer 2000) bestimmt (siehe Produktbeschreibung Firma MALVERN INSTRUMENTS GmbH, Herrenberg, Germany) Die Viskosität wurde nach der Brookfieldmethode (23 °C, Spindel 5, 10 Upm, mPas) ermittelt. Die Ergebnisse sind in Tabelle 1 zusammengefaßt.

**Tabelle 1: Kosmetische Zubereitungen (Mengenangaben in Gew.-% Aktivsubstanz - bezogen auf die Endzusammensetzung -)**

| **Zusammensetzung (INCI)** | **1** | **2** | **3** | **4** | **V1** | **V2** | **V3** | **V4** |
|---|---|---|---|---|---|---|---|---|
| **Wachskörper Cutina® AGS** | 25 | 25 | 25 | 25 | 25 | 25 | 25 | 25 |
| Ethylenglycol Distearate | | | | | | | | |
| **Cocoamidopropyl Betaine (a)** | 10 | 12 | 14 | 15 | 10 | 10 | 10 | 12 |
| **Cetiol® HE (c)** | 0,5 | 1,0 | 0,5 | 1,0 | - | 1,1 | 1,0 | 1,0 |
| PEG 7 Glyceryl cocoate | | | | | | | | |
| **Monomuls® 90-018 (b)** | - | 1,0 | - | 1,25 | - | - | 0,5 | 1,0 |
| Glyceryl Oleate | | | | | | | | |
| **Monomuls® 90-L12 (b)** | 1,5 | - | 2,5 | - | 1,3 | 0,9 | - | - |
| Glyceryl Laurate | | | | | | | | |
| **Cutina® GMS (b)** | - | 1,0 | - | 1,25 | - | - | 3,5 | 1,0 |
| Glyceryl Stearate | | | | | | | | |
| **Natriumlaurylethersulfat + 2 EO** | - | - | - | - | - | - | - | 2,5 |
| Glycerin | 5 | 5 | 5 | 5 | 5 | 5 | 5 | 5 |
| Benzoesäure | 0,5 | 0,5 | 0,5 | 0,5 | 0,5 | 0,5 | 0,5 | 0,5 |
| Wasser | ad 100 | | | | | | | |
| ***Gewichtsverhältnis a : b*** | 6,6:1 | 6:1 | 5,6:1 | 5:1 | 7,6:1 | 11:1 | 2,5:1 | 6:1 |
| ***Gewichtsverhältnis b : c*** | 3:1 | 2:1 | 4:1 | 2,5:1 | | 1,2:1 | 4:1 | 2:1 |
| ***Viskosität*** | 3000 | 4000 | 4000 | 5000 | 9000 | 5000 | 14000 | 16000 |
| ***Partikelgrößenverteilung*** | | | | | | | | |
| *< 15* µ***m*** | 99,9 | 98 | 98 | 99,9 | 58 | 76 | 68 | 55 |
| ***15-20*** µ***m*** | 0,1 | 2 | 2 | 0,1 | 35 | 21 | 24 | 35 |
| ***> 20*** µ***m*** | - | - | - | - | 7 | 3 | 8 | 10 |
| ***Durchschnittlicher Teilchendurchmesser*** | 5,5 | 6 | 6,5 | 5,0 | 14 | 13 | 15 | 16 |
| ***Aussehen*** | trüb | trüb | trüb | trüb | perlglanz | perlglanz | pedglanz | perlglanz |

## Patentansprüche

1. Trübungsmittelzubereitungen auf Basis von Wachskörpem, enthaltend
(a) mindestens ein amphoteres Tensid,
(b) mindestens ein Fettsäurepartialglycerid,
(c) mindestens ein Fettsäurepolyglycolester und gegebenenfalls
(d) ein Polyol
mit der Massgabe, dass das Gewichtsverhältnis von (a) : (b) zwischen 10 : 1 und 4 : 1, (b) : (c) zwischen 1 : 1 und 5 : 1 liegen und die Zubereitungen frei von Aniontensiden sind.

2. Mittel nach Anspruch 1, **dadurch gekennzeichnet, dass** sie als Komponente (a) amphotere Tenside enthalten, die ausgewählt sind aus der Gruppe, die gebildet wird von Alkylbetainen, Alkylamidobetainen, Aminopropionaten, Aminoglycinaten, Imidazoliniumbetainen und Sulfobetainen.

3. Mittel nach den Ansprüchen 1 und/oder 2, **dadurch gekennzeichnet, dass** sie als sie Komponente (a) Betaine der Formel **(I)** enthalten, 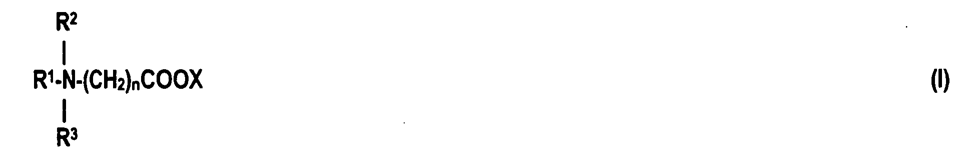 in der R¹ für Alkyl- und/oder Alkenylreste mit 6 bis 22 Kohlenstoffatomen, R² für Wasserstoff oder Alkylreste mit 1 bis 4 Kohlenstoffatomen, R³ für Alkylreste mit 1 bis 4 Kohlenstoffatomen, n für Zahlen von 1 bis 6 und X für ein Alkali- und/oder Erdalkalimetall oder Ammonium steht.

4. Mittel nach mindestens einem der Ansprüche 1 bis 3, **dadurch gekennzeichnet, dass** sie als Komponente (a) Betaine der Formel **(II)** enthalten, 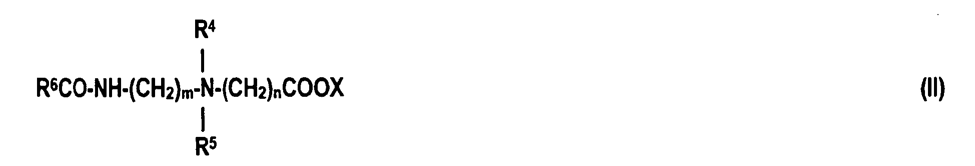 in der R⁶CO für einen aliphatischen Acylrest mit 6 bis 22 Kohlenstoffatomen und 0 oder 1 bis 3 Doppelbindungen, m für Zahlen von 1 bis 3 steht, R⁴ für Wasserstoff oder Alkylreste mit 1 bis 4 Kohlenstoffatomen, R⁵ für Alkylreste mit 1 bis 4 Kohlenstoffatomen, n für Zahlen von 1 bis 6 und X für ein Alkali- und/oder Erdalkalimetall oder Ammonium steht.

5. Mittel nach mindestens einem der Ansprüche 1 bis 4, **dadurch gekennzeichnet, dass** sie als Komponente (a) salzarme Betaine enthalten mit einem geringen Salzgehalt von maximal 13 Gew. % bezogen auf die Aktivsubstanz.

6. Mittel nach mindestens einem der Ansprüche 1 bis 5, **dadurch gekennzeichnet, dass** sie als Komponente (a) Betaine enthalten mit einem geringen Gehalt an freien Fettsäuren von maximal 4 Gew.% bezogen auf die Aktivsubstanz.

7. Mittel nach mindestens einem der Ansprüche 1 bis 6, **dadurch gekennzeichnet, dass** sie als Komponente (b) Fettsäurepartialglyceride der Formel **(IV)** enthalten, 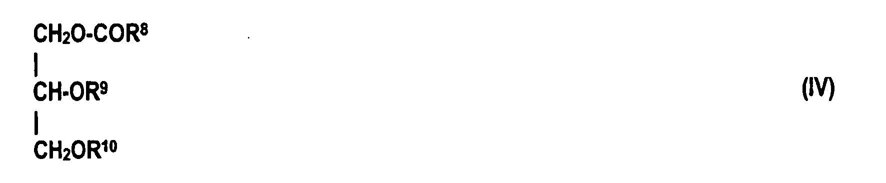 in der R⁸CO für einen linearen oder verzweigten, gesättigten und/oder ungesättigten Acylrest mit 6 bis 22, R⁹ und R¹⁰ unabhängig voneinander für R⁸CO oder OH und die Summe (m+n+p) für 0 oder Zahlen von 1 bis 100, vorzugsweise 5 bis 25 steht, mit der Maßgabe, daß mindestens einer der beiden Reste R⁹ und R¹⁰ OH bedeutet.

8. Mittel nach mindestens einem der Ansprüche 1 bis 7, **dadurch gekennzeichnet, dass** das Fettsäurepartialglycerid (b) einen Schmelzpunkt oberhalb 30°C aufweist.

9. Mittel nach mindestens einem der Ansprüche 1 bis 8, **dadurch gekennzeichnet, dass** der Fettsäurepolyolester (c) einen Schmelzpunkt unterhalb 25°C aufweist.

10. Mittel nach mindestens einem der Ansprüche 1 bis 9, **dadurch gekennzeichnet, dass** sie einen Feststoffgehalt von 20 bis 50 Gew.-% - bezogen auf die Endzusammensetzung - aufweisen.

11. Mittel nach mindestens einem der Ansprüche 1 bis 10, **dadurch gekennzeichnet, dass** sie die Komponenten (a) : (b) im Gewichtsverhältnis zwischen 7 : 1 bis 5 : 1 enthalten.

12. Mittel nach mindestens einem der Ansprüche 1 bis 11, **dadurch gekennzeichnet, dass** mindestens 85 % der Zusammensetzung Partikelgrössen kleiner als 15 µm aufweisen.

13. Verwendung von Zubereitungen nach Anspruch 1 als Trübungsmittel.

## Claims

1. Wax-based opacifier preparations containing
(a) at least one amphoteric surfactant,
(b) at least one fatty acid partial glyceride,
(c) at least one fatty acid polyglycol ester and optionally
(d) a polyol,
with the proviso that the ratio by weight of (a) to (b) is between 10:1 and 4:1 and the ratio by weight of (b) to (c) is between 1:1 and 5:1 and the preparations are free from anionic surfactants.

2. Preparations as claimed in claim 1, **characterized in that** they contain amphoteric surfactants selected from the group consisting of alkyl betaines, alkyl amidobetaines, aminopropionates, aminoglycinates, imidazolinium betaines and sulfobetaines as component (a).

3. Preparations as claimed in claims 1 and/or 2, **characterized in that** they contain betaines corresponding to formula **(I):** 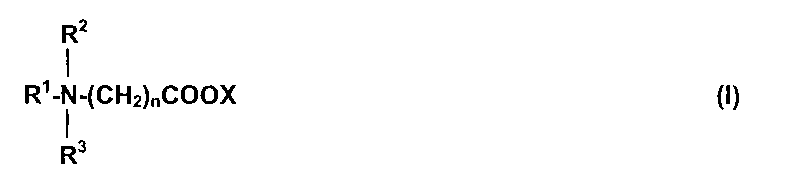 in which R¹ stands for alkyl and/or alkenyl groups containing 6 to 22 carbon atoms, R² stands for hydrogen or alkyl groups containing 1 to 4 carbon atoms, R³ stands for alkyl groups containing 1 to 4 carbon atoms, n is a number of 1 to 6 and X is an alkali metal and/or alkaline earth metal or ammonium,
as component (a).

4. Preparations as claimed in any of claims 1 to 3, **characterized in that** they contains betaines corresponding to formula **(II):** 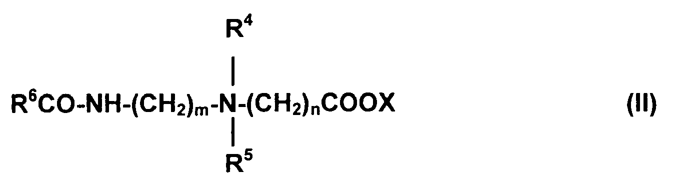 in which R⁶CO is an aliphatic acyl group containing 6 to 22 carbon atoms and 0 or 1 to 3 double bonds, m is a number of 1 to 3, R⁴ represents hydrogen or C₁₋₄ alkyl groups, R⁵ represents C₁₋₄ alkyl groups, n is a number of 1 to 6 and X is an alkali metal and/or alkaline earth metal or ammonium,
as component (a).

5. Preparations as claimed in at least one of claims 1 to 4, **characterized in that** they contain low-salt betaines with a low salt content of at most 13% by weight, based on active substance, as component (a).

6. Preparations as claimed in at least one of claims 1 to 5, **characterized in that** they contain betaines with a low content of free fatty acids of at most 4% by weight, based on active substance, as component (a).

7. Preparations as claimed in at least one of claims 1 to 6, **characterized in that** they contain fatty acid partial glycerides corresponding to formula **(IV):** 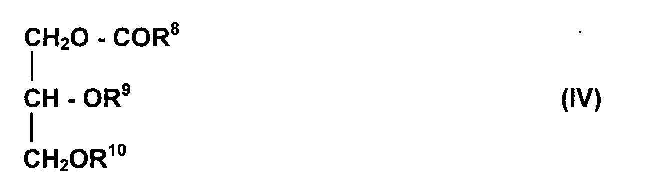 in which R⁸CO is a linear or branched, saturated and/or unsaturated acyl group containing 6 to 22 carbon atoms, R⁹ and R¹⁰ independently of one another have the same meaning as R⁸CO or represent OH and the sum (m+n+p) is 0 or a number of 1 to 100 and preferably 5 to 25, with the proviso that at least one of the two substituents R⁹ and R¹⁰ represents OH, as component (b).

8. Preparations as claimed in at least one of claims 1 to 7, **characterized in that** the fatty acid partial glyceride (b) has a melting point above 30°C.

9. Preparations as claimed in at least one of claims 1 to 8, **characterized in that** the fatty acid polyol ester (c) has a melting point below 25°C.

10. Preparations as claimed in at least one of claims 1 to 9, **characterized in that** they have a solids content of 20 to 50% by weight, based on the final composition.

11. Preparations as claimed in at least one of claims 1 to 10, **characterized in that** they contain components (a) and (b) in a ratio by weight of 7:1 to 5:1.

12. Preparations as claimed in at least one of claims 1 to 11, **characterized in that** at least 85% of the composition has particle sizes below 15 µm.

13. The use of the preparations claimed in claim 1 as opacifiers.

## Revendications

1. Préparations d'opacifiants à base de corps cireux, contenant
(a) au moins un tensioactif amphotère,
(b) au moins un glycéride partiel d'acides gras,
(c) au moins un ester de polyglycol avec un acide gras et éventuellement
(d) un polyol
étant entendu que le rapport pondéral de (a) : (b) est compris entre 10 : 1 et 4 : 1, de (b) : (c) est compris entre 1 : 1 et 5 : 1 et que les préparations sont exemptes de tensioactifs anioniques.

2. Opacifiants selon la revendication 1, **caractérisés en ce qu'**ils contiennent en tant que composant (a) des tensioactifs amphotères qui sont choisis dans le groupe qui est constitué par les alkylbétaïnes, les alkylamidobétaïnes, les aminopropionates, les aminoglycinates, les imidazoliniumbétaïnes et les sulfobétaïnes.

3. Opacifiants selon la revendication 1 et/ou la revendication 2, **caractérisés en ce qu'**ils contiennent en tant que composant (a) des bétaïnes de formule **(I)** 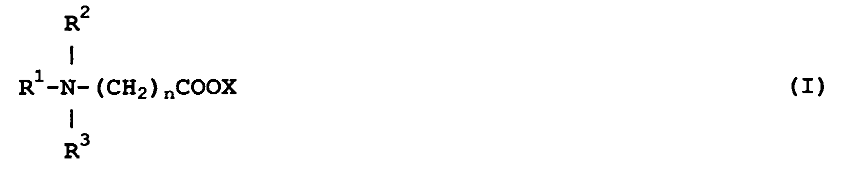 dans laquelle R¹ représente des radicaux alkyle et/ou alcényle ayant de 6 à 22 atomes de carbone, R² représente un atome d'hydrogène ou des radicaux alkyle ayant de 1 à 4 atomes de carbone, R³ représente des radicaux alkyle ayant de 1 à 4 atomes de carbone, n représente des nombres valant de 1 à 6 et X représente un métal alcalin et/ou un métal alcalino-terreux ou l'ion ammonium.

4. Opacifiants selon au moins l'une des revendications 1 à 3, **caractérisés en ce qu'**ils contiennent en tant que composant (a) des bétaïnes de formule **(II)** 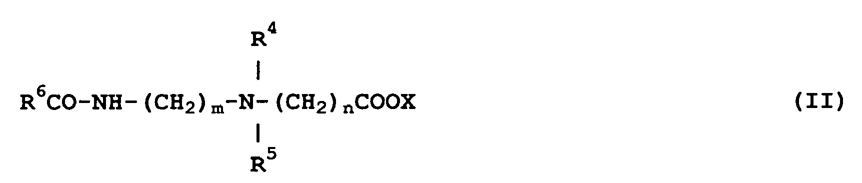 dans laquelle R⁶CO représente un radical acyle aliphatique ayant de 6 à 22 atomes de carbone et 0 ou 1 à 3 doubles liaisons, m représente des nombres valant de 1 à 3, R⁴ représente un atome d'hydrogène ou des radicaux alkyle ayant de 1 à 4 atomes de carbone, R⁵ représente des groupes alkyle ayant de 1 à 4 atomes de carbone, n représente des nombres valant de 1 à 6 et X représente un métal alcalin et/ou un métal alcalino-terreux ou l'ion ammonium.

5. Opacifiants selon au moins l'une des revendications 1 à 4, **caractérisés en ce qu'**ils contiennent en tant que composant (a) des bétaïnes pauvres en sel ayant une faible teneur en sel d'au maximum 13 % en poids, par rapport à la substance active.

6. Opacifiants selon au moins l'une des revendications 1 à 5, **caractérisés en ce qu'**ils contiennent en tant que composant (a) des bétaïnes ayant une faible teneur en acides gras libres d'au maximum 4 % en poids, par rapport à la substance active.

7. Opacifiants selon au moins l'une des revendications 1 à 6, **caractérisés en ce qu'**ils contiennent en tant que composant (b) des glycérides partiels d'acides gras de formule **(IV)** 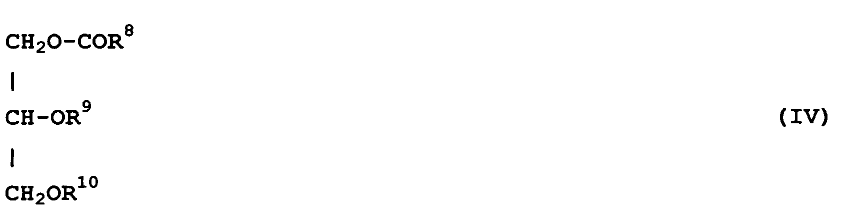 dans laquelle R⁸CO représente un radical acyle saturé et/ou insaturé, linéaire ou ramifié, ayant de 6 à 22 atomes de carbone, R⁹ et R¹⁰ représentent, indépendamment l'un de l'autre, R⁸CO ou OH et la somme (m+n+p) représente 0 ou des nombres valant de 1 à 100, de préférence de 5 à 25, étant entendu qu'au moins l'un des deux radicaux R⁹ et R¹⁰ représente OH.

8. Opacifiants selon au moins l'une des revendications 1 à 7, **caractérisés en ce que** le glycéride partiel d'acides gras (b) présente un point de fusion supérieur à 30 °C.

9. Opacifiants selon au moins l'une des revendications 1 à 8, **caractérisés en ce que** l'ester de polyol avec un acide gras (c) présente un point de fusion inférieur à 25 °C.

10. Opacifiants selon au moins l'une des revendications 1 à 9, **caractérisés en ce qu'**ils présentent une teneur en matière solide de 20 à 50 % en poids - par rapport à la composition finale.

11. Opacifiants selon au moins l'une des revendications 1 à 10, **caractérisés en ce qu'**ils contiennent les composants (a):(b) en un rapport molaire compris entre 7:1 et 5:1.

12. Opacifiants selon au moins l'une des revendications 1 à 11, **caractérisés en ce qu'**au moins 85 % de la composition présentent des tailles de particule inférieures à 15 µm.

13. Utilisation des préparations selon la revendication 1 en tant qu'opacifiants.
